(19) Europäisches Patentamt / European Patent Office / Office européen des brevets

(11) **EP 2 441 420 B1**

(12) **EUROPEAN PATENT SPECIFICATION**

(45) Date of publication and mention
of the grant of the patent:
**24.12.2014 Bulletin 2014/52**

(21) Application number: **10786166.8**

(22) Date of filing: **08.06.2010**

(51) Int Cl.:
*A61F 13/49* (2006.01)  *A61F 13/53* (2006.01)
*A61F 13/534* (2006.01)  *A61F 13/535* (2006.01)
*A61F 13/537* (2006.01)  *A61F 13/15* (2006.01)
*A61F 13/539* (2006.01)

(86) International application number:
**PCT/JP2010/059708**

(87) International publication number:
**WO 2010/143635 (16.12.2010 Gazette 2010/50)**

(54) **WATER ABSORBENT SHEET**

WASSERABSORBIERENDE PLATTE

FEUILLE HYDRO-ABSORBANTE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **12.06.2009 JP 2009141365**

(43) Date of publication of application:
**18.04.2012 Bulletin 2012/16**

(73) Proprietor: **Sumitomo Seika Chemicals Co., Ltd. Hyogo 675-0145 (JP)**

(72) Inventors:
• **UEDA, Koji**
**Hyogo 672-8076 (JP)**
• **HINAYAMA, Tetsuhiro**
**Hyogo 672-8076 (JP)**
• **HANDA, Masayoshi**
**Hyogo 672-8076 (JP)**
• **TSUNO, Syuji**
**Hyogo 675-0145 (JP)**

(74) Representative: **Duckworth, Timothy John**
**J A Kemp**
**14 South Square**
**Gray's Inn**
**London WC1R 5JJ (GB)**

(56) References cited:
EP-A1- 2 111 835  WO-A1-2005/087162
FR-A1- 2 364 024  JP-A- 10 118 117
JP-A- 2004 275 225  JP-A- 2006 280 753
JP-U- H0 659 039  US-A- 5 865 822
US-A- 5 895 379  US-A1- 2003 163 106
US-A1- 2007 197 987

**Description**

TECHNICAL FIELD

The present invention relates to a thin water-absorbent sheet which can be used in the fields of hygienic materials and the like. More specifically, the present invention relates to a water-absorbent sheet containing a very small amount of pulp, which can be suitably used in absorbent articles, such as disposable diapers and incontinence pads, having high absorbent properties even when being thin. In addition, the present invention relates to an absorbent article using the water-absorbent sheet. Further, the present invention relates to a method of efficiently producing the above-mentioned water-absorbent sheet with stable properties.

BACKGROUND ART

Body liquid absorbent articles represented by disposable diapers or the like have a structure in which an absorbent material for absorbing a liquid such as a body liquid is sandwiched with a flexible liquid-permeable surface sheet (top sheet) positioned on a side contacting a body and a liquid-impermeable backside sheet (back sheet) positioned on a side opposite to that contacting the body.

Conventionally, there have been increasing demands for thinning and light-weighing of absorbent articles, from the viewpoint of designing property and convenience upon carrying, and efficiency upon distribution. Further, in the recent years, there have been growing needs for so-called eco-friendly intentions, in which resources are effectively utilized so that use of natural materials that require a long time to grow such as trees is avoided as much as possible, from the viewpoint of environmental protection. Conventionally, a method for thinning that is generally carried out in absorbent articles is a method of reducing hydrophilic fibers such as disintegrated pulp of a wood material, which has a role of fixing a water-absorbent resin in an absorbent material, while increasing a water-absorbent resin.

An absorbent material in which a water-absorbent resin is used in a large amount with a lowered proportion of a hydrophilic fiber is preferred in thinning, from the viewpoint of reducing bulky hydrophilic fibers while retaining a liquid. However, when distribution or diffusion of a liquid upon actually using in an absorbent article such as disposable diapers is considered, there is a disadvantage that if a large amount of the water-absorbent resin is formed into a soft gel-like state by absorption, a so-called "gel-blocking phenomenon" takes place, whereby liquid diffusibility is markedly lowered and a liquid permeation rate of the absorbent material is slowed down. This "gel-blocking phenomenon" is a phenomenon in which especially when an absorbent material in which water-absorbent resins are highly densified absorbs a liquid, water-absorbent resins existing near a surface layer absorb the liquid to form soft gels that are even more densified near the surface layer, so that a liquid permeation into an internal of an absorbent material is inhibited, thereby making the internal of the water-absorbent resin incapable of efficiently absorbing the liquid.

In view of the above, conventionally, as a means of inhibiting gel blocking phenomenon which takes place by reducing hydrophilic fibers while using a water-absorbent resin in a large amount, for example, proposals such as a method using an absorbent polymer having such properties as specified Saline Flow Conductivity and Performance under Pressure (see Patent Publication 1), and a method using a water-absorbent resin prepared by heat-treating a specified water-absorbent resin precursor with a specified surface crosslinking agent (see Patent Publication 2) have been made.

However, in these methods, the absorption properties as absorbent materials in which water-absorbent resins are used in large amounts are not satisfactory. In addition, there arise some problems that the water-absorbent resin is subjected to be mobile before use or during use because hydrophilic fibers that play a role of fixing the water-absorbent resin are reduced. The absorbent material in which the localization of the absorbent resin takes place is more likely to cause gel-blocking phenomenon.

Further, in an absorbent material of which hydrophilic fibers that contribute to retention of the form are reduced has a lowered strength as an absorbent material, deformation such as twist-bending or tear before or after the absorption of a liquid is likely to take place. In an absorbent material with deformation, liquid diffusibility has markedly lowered, so that abilities inherently owned by the absorbent material cannot be exhibited. In order to try to avoid such phenomena, a ratio of hydrophilic fibers and a water-absorbent resin would be limited, thereby posing limitations in the thinning of an absorbent article.

In view of the above, in recent years, as a next generation style absorbent material which is capable of increasing a content of a water-absorbent resin while using hydrophilic fibers in an absorbent material as little as possible, studies have been widely made on an absorbent laminate that substantially does not contain hydrophilic fibers in an absorbent layer, a water-absorbent sheet or the like. For example, a method using an absorbent laminate comprising two pieces of nonwoven fabrics, and a reticular layer comprising two, upper and lower layers of hot melt adhesives provided between the nonwoven fabrics, in which the nonwoven fabrics are bonded with the reticular layer (see Patent Publication 3), and the like.

However, in a case where hydrophilic fibers are hardly used, the gel blocking phenomenon as mentioned above are likely to take place. Even in a case where gel blocking phenomenon does not take place, a thing that would serve the role of conventional hydrophilic fibers by which a body fluid such as urine is temporarily subjected to water retention and diffusion of the liquid to an overall absorbent material is lacking, so that a liquid leakage is likely to occur in the absorbent laminate, without being able to sufficiently capture the liquid.

Further, when an adhesive is used for retaining the shape of an absorbent laminate, the surface of an absorbent resin is coated with an adhesive, so that absorbent properties are likely to be lowered. Alternatively, an upper side and a lower side of nonwoven fabrics are firmly adhered with an adhesive to confine an water-absorbent resin in a pouched form or the like, so that the absorption properties inherently owned by the water-absorbent resin are less likely to be exhibited.

When adhesive strength of an absorbent laminate is weakened in order to improve absorption properties, not only a large amount of the absorbent resin is detached upon handling the laminate, thereby making unfavorable economically, but also the laminate is exfoliated due to deficiency in strength, so that there are some possibilities of loss of commercial values. In other words, if adhesion is strengthened, gel blocking phenomenon or liquid leakage occurs, and if adhesion is weakened, the detachment of a water-absorbent resin and the breaking of the laminate take place, so that an absorbent laminate or a water-absorbent sheet having satisfactory properties is not obtained.

There is also a method of immobilizing a water-absorbent resin to a substrate without using an adhesive, which is, for example, a method of adhering water-absorbent polymer particles in the process of polymerization to a synthetic fibrous substrate to carry out polymerization on the fibrous substrate (see Patent Publication 4), a method of polymerizing a monomer aqueous composition containing acrylic acid and an acrylic acid salt as main components on a nonwoven fabric substrate by means of electron beam irradiation (see Patent Publication 5), and the like.

In these methods, while the synthetic fibrous substrate is penetrated into the polymer particles to be firmly adhered, there are some disadvantages that it is difficult to complete the polymerization reaction in the substrate, so that unreacted monomers and the like remain in the substrate in large amounts.

In addition, a laminate having a 5-layered structure in which homogeneity is improved and a water-absorbent resin is effectively utilized is disclosed (see Patent Publication 6). The laminate might be effective for a trace amount of liquid (test solution: 0.2 cc); however, not only a total amount of the water-absorbent resin used is small but also a water-absorbent resin in a layer near human body (first absorbent layer) is in a relatively small amount; therefore, when an amount of liquid such as urine or blood is large, the amount of re-wet becomes large, thereby having a disadvantage of increased unpleasant feel.

In addition, a technique of folding an absorbent material to be used in an absorbent article or the like is being studied, including, for example a method including allowing a body liquid to flow in an internal part via groves formed by folding an absorbent material, and increasing an area to be absorbed (Patent Publication 7); a method of increasing an area to be absorbed utilizing a bending structure comprising an absorbent material being folded (Patent Publication 8); and the like.

These methods are techniques of forming channels and pleats on a side to which a liquid to be absorbed is supplied, thereby utilizing a force of quickly sponging a liquid, owned by hydrophilic fibers such as absorption paper or disintegrated pulp. The absorbent material having the constitution might be acknowledged to show improvements in the properties in cases of conventional articles where a ratio of hydrophilic fibers is high; however, in thin style absorbent materials having a lowered ratio of hydrophilic fibers and an increased ratio of an absorbent resin, which are of the recent development trends, a liquid to be absorbed flows along channels and pleats before being absorbed, thereby leading to leakage, so that the absorbent articles are not necessarily likely to exhibit sufficient effects in the properties of the water absorption capacity.

US 5895379 relates to an absorbent core comprising an upper fluid acquisition/distribution component, at least one upper fluid storage component, a fluid acquisition zone and a lower fluid acquisition/distribution component. US 2003/163106 describes an absorbent article including a liquid-permeable top layer, an absorbent core layer, and a breathable outer cover, which article has a breathable inner laminate between the absorbent core and the outer cover. FR 2364024 describes disposable absorbent articles that have an intermediate layer treated with a surfactant. US 2007/197987 relates to a method of making an absorbent composite that includes a first fabric, a second fabric, and particles positioned between the two fabrics and absorbent articles made from the absorbent composite.

PRIOR ART PUBLICATION

PATENT PUBLICATIONS

Patent Publication 1: Japanese Unexamined Patent Publication No. Hei-9-510889

Patent Publication 2: Japanese Patent Laid-Open No. Hei-8-57311
Patent Publication 3: Japanese Patent Laid-Open No. 2000-238161
Patent Publication 4: Japanese Patent Laid-Open No. 2003-11118
Patent Publication 5: Japanese Patent Laid-Open No. Hei-02-048944
Patent Publication 6: Japanese Utility Model Laid-Open No. Hei-6-059039
Patent Publication 7: Japanese Patent Laid-Open No. Hei-9-313530
Patent Publication 8: Japanese Patent Laid-Open No. 2002-345871

## SUMMARY OF THE INVENTION

## PROBLEMS TO BE SOLVED BY THE INVENTION

An object of the present invention is to provide a water-absorbent sheet which is capable of accomplishing thinning while avoiding gel blocking phenomenon, irrespective of having a large content of a water-absorbent resin, while obtaining fundamental properties (high strength, fast liquid permeation rate, small amount of re-wet, and small liquid leakage) as a water-absorbent sheet at a high level, even for a water-absorbent sheet containing a very small amount of pulps. Another object of the present invention is to provide a method for producing a water-absorbent sheet having high fundamental properties in a stable and efficient manner.

## MEANS TO SOLVE THE PROBLEMS

Specifically, the present invention relates to:

[1] a water-absorbent sheet comprising a structure in which an absorbent layer containing a water-absorbent resin and an adhesive is sandwiched with a hydrophilic nonwoven fabric, wherein the water-absorbent sheet has a structure in which the absorbent layer is fractionated into a primary absorbent layer and a secondary absorbent layer with a substrate layer formed by laminating two or more layers of a substrate having breathability adhered together with an adhesive, the substrate layer having a basis weight of 25 $g/m^2$ to 250 $g/m^2$; and wherein the water-absorbent sheet is obtained by folding the front face side of a water-absorbent sheet precursor and adhering the substrate formed in two layers with each other with an adhesive in an amount of from 0.1 to 50 $g/m^2$, the water-absorbent sheet precursor comprising an absorbent layer comprising a water-absorbent resin and an adhesive, sandwiched between a substrate having a breathability in a front face side and a hydrophilic nonwoven fabric in a back face side; wherein the substrate is made of at least one member selected from the group consisting of sanitary paper, porous films, cellulose-containing synthetic fiber nonwoven fabric, rayon-containing synthetic fiber nonwoven fabric, and hydrophilically treated synthetic fiber nonwoven fabric;
[2] an absorbent article comprising the water-absorbent sheet as defined in the above [1], sandwiched between a liquid-permeable sheet and a liquid-impermeable sheet and
[3] a method for producing a water-absorbent sheet comprising the steps of:

preparing a water-absorbent sheet precursor comprising an absorbent layer comprising a water-absorbent resin and an adhesive, sandwiched between a substrate having a breathability in a front face side and a hydrophilic nonwoven fabric in a back face side; and
folding the water-absorbent sheet precursor in the front face side, and adhering the substrate formed in two layers with each other with an adhesive in an amount of from 0.1 to 50 $g/m^2$,

thereby giving a water-absorbent sheet comprising a substrate layer having a total basis weight of the substrate of 25 $g/m^2$ to 250 $g/m^2$, the substrate layer being formed by laminating two or more layers of a substrate, wherein the water-absorbent sheet comprises an absorbent layer separated into a primary absorbent layer and a secondary absorbent layer with the substrate layer; wherein the substrate is made of at least one member selected from the group consisting of sanitary paper, porous films, cellulose-containing synthetic fiber nonwoven fabric, rayon-containing synthetic fiber nonwoven fabric, and hydrophilically treated synthetic fiber nonwoven fabric.

## EFFECTS OF THE INVENTION

The water-absorbent sheet of the present invention exhibits some excellent effects that the water-absorbent sheet is capable of accomplishing thinning and avoidance of gel blocking phenomenon and liquid leakage, while obtaining basic properties as a water-absorbent sheet at a high level, even for a water-absorbent sheet containing a very small amount of pulps. Further, the method for producing a water-absorbent sheet of the present invention exhibits some effects that the water-absorbent sheet of the present invention can be efficiently produced, and that the resulting water-absorbent sheet has smaller variances of the properties.

BRIEF DESCRIPTION OF THE DRAWINGS

[Figure 1] Figure 1 is an enlarged cross-sectional view schematically showing a structure of one embodiment of a water-absorbent sheet of the present invention.
[Figure 2] Figure 2 is an enlarged cross-sectional view schematically showing a structure of one embodiment of a water-absorbent sheet of the present invention.
[Figure 3] Figure 3 is a plan view showing spreading positions of a water-absorbent resin and/or an adhesive, and folding points, in the method of the present invention.
[Figure 4] Figure 4 is a plan view showing spreading positions of a water-absorbent resin and/or an adhesive, and folding points, in the method of the present invention.
[Figure 5] Figure 5 is a schematic view showing arrangements of a water-absorbent sheet and an acrylic plate, for evaluating strength of the water-absorbent sheet.
[Figure 6] Figure 6 is a schematic view of an apparatus used for carrying out a slope leakage test.
[Figure 7] Figure 7 is an enlarged cross-sectional view schematically showing a structure of a water-absorbent sheet of a comparative example in the present invention.

MODES FOR CARRYING OUT THE INVENTION

The water-absorbent sheet of the present invention has a structure in which an absorbent layer containing a water-absorbent resin and an adhesive is sandwiched with hydrophilic nonwoven fabrics, wherein the water-absorbent sheet has a structure in which the absorbent layer is fractionated into a primary absorbent layer and a secondary absorbent layer with a substrate layer having a specified constitution, so that the absorbent layers do not substantially contain hydrophilic fibers such as pulps that contribute to fixation of a water-absorbent resin in the absorbent layers, and shape retention of the absorbent layer, and whereby the water-absorbent sheet obtained is thin and has high-performance, with a very small amount of pulps used.

As the kinds of the water-absorbent resins, commercially available water-absorbent resins can be used. For example, the water absorbent resin includes hydrolysates of starch-acrylonitrile graft copolymers, neutralized products of starch-acrylic acid graft polymers, saponified products of vinyl acetate-acrylic acid ester copolymers, partially neutralized products of polyacrylic acid, and the like. Among them, the partially neutralized products of polyacrylic acids are preferred, from the viewpoint of production amount, production costs, water absorbency of product, and the like. Methods for synthesizing partially neutralized products of polyacrylic acid include reversed phase suspension polymerization method and aqueous solution polymerization method. Among them, the water-absorbent resins obtained according to reversed phase suspension polymerization method are more preferably used, from the viewpoint of excellent flowability of the resulting particles, smaller amounts of fine powder, high water-absorbent properties, such as water absorption capacity and water-absorption rate.

The partially neutralized product of a polyacrylic acid has a degree of neutralization of preferably 50% by mol or more, and even more preferably from 70 to 90% by mol, from the viewpoint of increasing osmotic pressure of the water-absorbent resin, thereby increasing water absorbency.

The water-absorbent resin is contained, in a total amount of a primary absorbent layer and a secondary absorbent layer, in the water-absorbent sheet of preferably from 100 to 1000 g per one square meter of the water-absorbent sheet, i.e. 100 to 1000 $g/m^2$, more preferably from 150 to 800 $g/m^2$, even more preferably from 200 to 700 $g/m^2$, and most preferably from 250 to 600 $g/m^2$, from the viewpoint of obtaining sufficient water-absorption ability even when a water-absorbent sheet of the present invention is used for an absorbent article. The water-absorbent resin is contained in an amount of preferably 100 $g/m^2$ or more, from the viewpoint of exhibiting sufficient water absorption ability as a water-absorbent sheet, thereby suppressing re-wetting, and the water-absorbent resin is contained in an amount of preferably 1000 $g/m^2$ or less, from the viewpoint of suppressing the generation of gel blocking phenomenon, exhibiting liquid diffusibility as a water-absorbent sheet, and further improving a liquid permeation rate.

The resin ratio (mass ratio) of the primary absorbent layer/secondary absorbent layer is preferably within the range of from primary absorbent layer/secondary absorbent layer = 95/5 to 55/45, more preferably with the range of from primary absorbent layer/secondary absorbent layer = 95/5 to 60/40, even more preferably within the range of from primary absorbent layer/secondary absorbent layer = 95/5 to 70/30, and most preferably within the range of from primary absorbent layer/secondary absorbent layer = 90/10 to 80/20. The primary absorbent layer/secondary absorbent layer is in a ratio of preferably 95/5 or less, from the viewpoint of sufficiently exhibiting water absorbency of a secondary absorbent layer, thereby preventing liquid leakage, and the primary absorbent layer/secondary absorbent layer is preferably in a ratio of 55/45 or more, from the viewpoint of increasing dry feel of the primary absorbent layer and lowering the amount of re-wet after liquid absorption.

The absorbency of the water-absorbent sheet of the present invention is influenced by the water absorbency of the water-absorbent resin used. Therefore, it is preferable that the water-absorbent resin to be used in the present invention is those selected with optimal ranges in water absorption properties such as water absorption capacity (water-retention capacity), and water absorption rate of the water-absorbent resin, by taking the constitution of each component of the water-absorbent sheet or the like into consideration. Therefore, in the water-absorbent resin used in the present invention, the kinds of the water-absorbent resin of the primary absorbent layer and the kinds of the water-absorbent resin of the secondary absorbent layer may be identical to or different from each other.

In the present specification, the water-retention capacity of the water-absorbent resin is evaluated as a water-retention capacity of saline solution. The water-absorbent resin has a water-retention capacity of saline solution of preferably 25 g/g or more, more preferably from 25 to 60 g/g, and even more preferably from 30 to 50 g/g, from the viewpoint of absorbing a liquid in a larger amount, and preventing the gel blocking phenomenon while keeping the gel strong during absorption. The water-retention capacity of saline solution of the water-absorbent resin is a value obtainable by a measurement method described in Examples set forth below.

In the present specification, the water absorption rate of the water-absorbent resin is evaluated as a water absorption rate of saline solution. The water-absorbent resin has a water-absorption rate of saline solution of preferably from 2 to 70 s, more preferably from 3 to 60 s, and even more preferably from 3 to 55 s, from the viewpoint of speeding up the permeation rate of the water-absorbent sheet of the present invention, thereby preventing a liquid leakage upon use in an absorbent article. The water-absorption rate of the water-absorbent resin as used herein is a value obtainable by a measurement method described in Examples set forth below.

In the water-absorbent sheet of the present invention, it is preferable that there is a positive difference in values between the water absorption rate of saline solution of a water-absorbent resin in the primary absorbent layer and the rate of that of the secondary absorbent layer. The greater the difference therebetween, effects of avoiding the stagnation of a liquid in the primary absorbent layer, to thereby increase dry feel, and preventing a liquid leakage are even more strongly exhibited. Specifically, (the rate of resin in the primary absorbent layer) - (the rate of resin in the secondary absorbent layer) is preferably 10 seconds or more, more preferably 15 seconds or more, and even more preferably 20 seconds or more.

The water-absorbent resin has a median particle size of preferably from 100 to 600 $\mu$m, more preferably from 150 to 550 $\mu$m, and even more preferably from 200 to 500 $\mu$m, from the viewpoint of preventing the scattering of the water-absorbent resin in the water-absorbent sheet, the gel blocking phenomenon during water absorption, and at the same time reducing the rugged feel of the water-absorbent sheet, thereby improving texture.

The adhesive used in the absorbent layer includes, for example, rubber adhesives such as natural rubbers, butyl rubbers, and polyisoprene; styrenic elastomer adhesives such as styrene-isoprene block copolymers (SIS), styrene-butadiene block copolymers (SBS), styrene-isobutylene block copolymers (SIBS), and styrene-ethylene-butylene-styrene block copolymers (SEBS); ethylene-vinyl acetate copolymer (EVA) adhesives; ethylene-acrylic acid derivative copolymer adhesives such as ethylene-ethyl acrylate copolymer (EEA), and ethylene-butyl acrylate copolymer (EBA); ethylene-acrylic acid copolymer (EAA) adhesives; polyamide adhesives such as copolymer nylons and dimer acids-based polyamides; polyolefin adhesives such as polyethylenes, polypropylenes, atactic polypropylenes, and copolymeric polyolefins; polyester adhesives such as polyethylene terephthalate (PET), polybutylene terephthalate (PBT), and copolymeric polyesters; and acrylic adhesives, and these adhesives may be used together in two or more kinds. In the present invention, the ethylene-vinyl acetate copolymer adhesives, the styrenic elastomer adhesives, the polyolefinic adhesives, and the polyester adhesives are preferred, from the viewpoint of high adhesive strength, thereby making it possible to prevent exfoliation of a hydrophilic nonwoven fabric and scattering of the water-absorbent resin in the water-absorbent sheet.

The adhesive has a melting temperature or a softening point of preferably from 60° to 180°C, and more preferably from 70° to 150°C, from the viewpoint of sufficiently fixing a water-absorbent resin to a nonwoven fabric, and at the same time preventing thermal deterioration or deformation of the nonwoven fabric. Here, in the water-absorbent sheet of the present invention, in the process of producing a water-absorbent sheet, after melting, the adhesive is adhered to a nonwoven fabric or a hydrophilic resin in a solid state by cooling the molten adhesive.

The adhesive in the absorbent layer in the water-absorbent sheet is contained in an amount preferably in the range of from 0.05 to 2.0 times, more preferably in the range of from 0.08 to 1.5 times, and even more preferably in the range of from 0.1 to 1.0 time the amount of the water-absorbent resin contained (mass basis). It is preferable that the adhesive is contained in an amount of 0.05 times or more, from the viewpoint of having sufficient adhesion, thereby preventing exfoliation of the hydrophilic nonwoven fabrics themselves or scattering of the water-absorbent resin, and the localization of the water-absorbent resin in the absorbent layers upon folding, and increasing strength of a water-absorbent sheet. It is preferable that the adhesive is contained in an amount of 2.0 times or less, from the viewpoint of avoiding the inhibition of the swelling of the water-absorbent resin due to too strong adhesion to each other, thereby improving a permeation rate or liquid leakage of a water-absorbent sheet.

The hydrophilic nonwoven fabric is not particularly limited, as long as the hydrophilic nonwoven fabric is a known nonwoven fabric in the field of art. The hydrophilic nonwoven fabric includes nonwoven fabrics made of polyolefin fibers such as polyethylene (PE) and polypropylene (PP); polyester fibers such as polyethylene terephthalate (PET), polytrimethylene terephthalate (PTT), and polyethylene naphthalate (PEN); polyamide fibers such as nylon; rayon fibers, and other synthetic fibers; nonwoven fabrics produced by mixing cotton, silk, hemp, pulp (cellulose) fibers, or the like, from the viewpoint of liquid permeability, flexibility and strength upon forming into a sheet, and the hydrophilic nonwoven fabric may be a mixture of two or more kinds of fibers. In addition, its surface may be subjected to a hydrophilic treatment according to a known method, as occasion demands. The nonwoven fabric made of synthetic fibers is preferably used, from the viewpoint of increasing the strength of the water-absorbent sheet, and especially at least one member selected from the group consisting of rayon fibers, polyolefin fibers, polyester fibers, and mixtures thereof is preferred. The hydrophilic nonwoven fabric made of synthetic fibers may contain pulp fibers in a small amount to an extent that the thickness of the water-absorbent sheet would not increase.

The hydrophilic nonwoven fabric is preferably a nonwoven fabric having an appropriate basis weight and an appropriate thickness, from the viewpoint of giving the water-absorbent sheet of the present invention excellent liquid permeability, flexibility, strength and cushioning property, and speeding up the permeation rate of the water-absorbent sheet. The hydrophilic nonwoven fabric has a basis weight of preferably 15 $g/m^2$ or more, more preferably in the range of from 25 to 250 $g/m^2$, and even more preferably in the range of from 35 to 150 $g/m^2$. Also, the hydrophilic nonwoven fabric has a thickness of preferably in the range of from 200 to 1500 $\mu$m, more preferably in the range of from 250 to 1200 $\mu$m, and even more preferably in the range of from 300 to 1000 $\mu$m.

The substrate layer used in the present invention is a substrate layer formed by laminating two or more layers of a substrate having breathability adhered together with an adhesive, wherein the substrate layer satisfies the following requirements (1) and (2): (1) the substrate layer having a basis weight of 25 $g/m^2$ to 250 $g/m^2$; and (2) the substrates having breathability are adhered therebetween with an adhesive in an amount of from 0.1 to 50 $g/m^2$. Since a specified substrate layer as described above is provided, the above-mentioned problem of realizing thinning of a water-absorbent sheet while avoiding the gel block phenomenon can be solved.

It is necessary that the substrate layer fractionating the absorbent layer into the primary absorbent layer and the secondary absorbent layer is a material which appropriately permeates a liquid absorbed and appropriately diffuses in the substrate. Moreover, as the function of a substrate layer, if water permeability is weighed with importance, gel blocking occurs, and if diffusion is weighed with importance, liquid leakage occurs, so that it is necessary to find a material with a proper balance to be used as a water-absorbent sheet. The present inventors have found that the substrate layer made of the constitution mentioned above remarkably enhances the properties of the water-absorbent sheet, and the present invention is completed thereby.

The materials constituting the substrate layer are at least one member selected from the group consisting of sanitary papers, porous films, nonwoven fabrics made of cellulose-containing synthetic fibers, nonwoven fabrics made of rayon-containing synthetic fibers, and nonwoven fabrics made of hydrophilically treated synthetic fibers.

The sanitary papers include, for examples, tissue paper, toilet paper, paper towel, and the like. The porous films include perforated films having numerous circular pores on films made of polyethylene and polypropylene. The nonwoven fabrics made of cellulose-containing synthetic fibers include, for example, airlaid nonwoven fabrics made of pulp/PET/polyethylene (PE), pulp/PET/polypropylene (PP), pulp/PE/PP. The nonwoven fabrics made of rayon-containing synthetic fibers include, for example, spun lace nonwoven fabrics made of rayon/PET, rayon/PE, or rayon/PET/PE. The nonwoven fabrics made of hydrophilically treated synthetic fibers include, for example, an air-through nonwoven fabric of a polyolefin comprising PE, PP, or PE/PP coated with a hydrophilic surfactant such as a fatty acid ester-type nonionic surfactant or a polyglycerol fatty acid ester. Among them, the nonwoven fabrics made of rayon-containing synthetic fibers and the nonwoven fabrics made of hydrophilically treated synthetic fibers are more preferably used, from the viewpoint of strength and availability and the like of the resulting water-absorbent sheet. Further, the nonwoven fabrics made of rayon-containing synthetic fibers are most preferably used, from the viewpoint of the properties of the resulting water-absorbent sheet.

The basis weight of each of the substrates is designed so that the substrates constituting the substrate layer has a total basis weight of 25 $g/m^2$ to 250 $g/m^2$, more preferably in the range of from 30 to 250 $g/m^2$, and even more preferably in the range of from 35 to 150 $g/m^2$. The thickness of the substrate is not particularly limited, and the thickness is preferably from 150 to 1500 $\mu$m, more preferably from 200 to 1000 $\mu$m, and even more preferably from 250 to 800 $\mu$m. The substrate preferably has a thickness of 1500 $\mu$m or less, from the viewpoint of thinning a water-absorbent sheet, and on the other hand, the substrate preferably has a thickness of 150 $\mu$m or more, from the viewpoint of obtaining sufficient strength against stretching and twisting during the production and upon use of a water-absorbent sheet.

The adhesive for adhering a substrate forming a substrate layer can be the same ones as those adhesives used in the absorbent layer mentioned above. Among them, in the present invention, the ethylene-vinyl acetate copolymer adhesives, the styrenic elastomer adhesives, the polyolefin adhesives, and the polyester adhesives are preferred, from the viewpoint of having a strong adhesive strength, thereby capable of preventing the substrates themselves from being exfoliated.

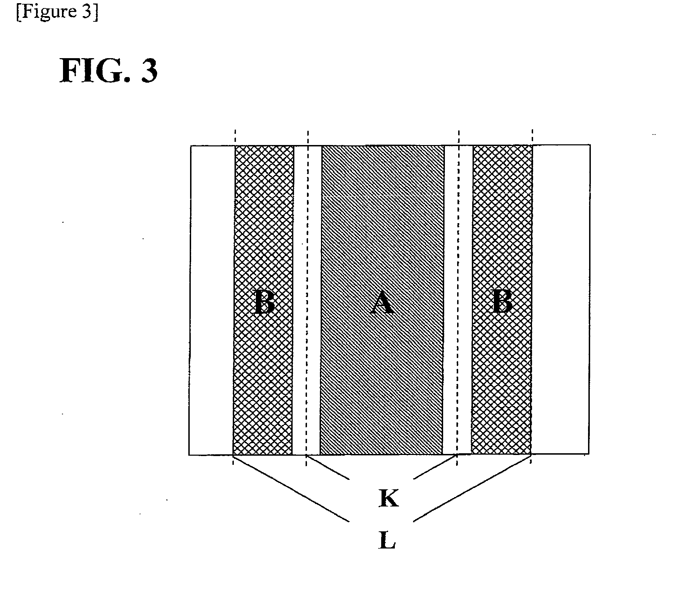

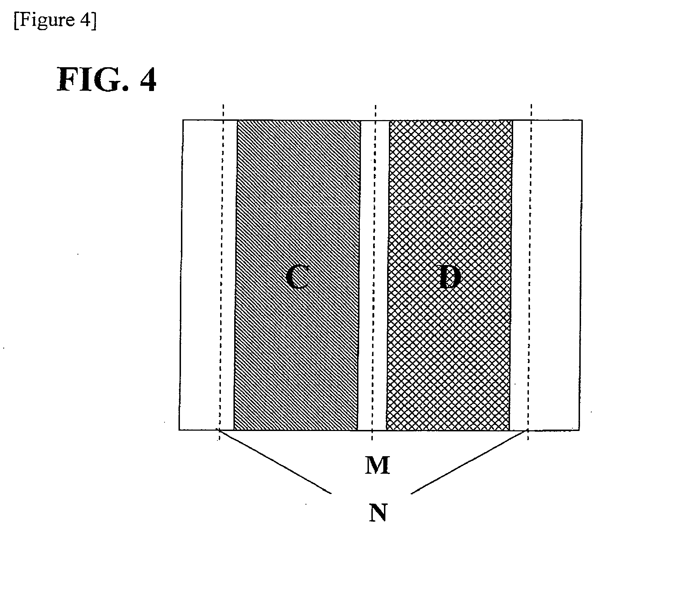

In the present invention, a space between the substrates forming a substrate layer is appropriately adhered with an adhesive. Here, the amount of the adhesive between the laminated substrates is preferably in the range of from 0.1 to 50 g (in other words, 0.1 to 50 g/m$^2$), per unit square meter of an area of an absorbent layer (for example, regions A and B in Figure 3, and regions C and D in Figure 4), more preferably in the range of from 0.5 to 35 g/m$^2$, and even more preferably from 1 to 25g/m$^2$. The adhesive is contained in an amount of preferably 0.1 g/m$^2$ or more, from the viewpoint of sufficient adhesion of the substrates themselves, so that the strength of the water-absorbent sheet is increased, and the adhesive is contained in an amount of preferably 50 g/m$^2$ or less, from the viewpoint of avoiding excessive adhesion between the substrates, thereby keeping an appropriate balance between diffusion and permeation of the liquid, and improving a permeation rate and a liquid leakage of the water-absorbent sheet.

In a case where a powdery solid adhesive is mixed with a water-absorbent resin and dispersed, an adhesive having a median particle size of the same range as the median particle size of the above-mentioned water-absorbent resin is preferably used, from the viewpoint of homogeneity of the mixture. On the other hand, in a case of an adhesive used in a liquid state by a heating-fusing means or the like, when the adhesive upon coating has a thin line width, it is unfavorable because the coating of the adhesive becomes too dense, thereby inhibiting diffusion and permeation of the liquid; and when the adhesive has a thick line width, the adhesive is sparsely applied, and thereby the exfoliation of the substrate layer is more likely to take place. From these viewpoints, the liquid upon coating a substrate has a line width of preferably from 20 to 200 μm, and more preferably from 40 to 120 μm.

In addition, the water-absorbent sheet of the present invention may properly be formulated with an additive such as a deodorant, an antibacterial agent, or a gel stabilizer.

The water-absorbent sheet of the present invention has one feature in the aspect of enabling thinning of the sheet. When the use in absorbent articles is taken into consideration, the water-absorbent sheet has a thickness, on a dry basis, of preferably 5 mm or less, more preferably from 0.5 to 4 mm, and even more preferably from 1 to 3 mm.

Further, the water-absorbent sheet of the present invention has one feature in that a liquid has a fast permeation rate, and the water-absorbent sheet has a total permeation rate of preferably 100 seconds or less, more preferably 90 seconds or less, and even more preferably 80 seconds or less, when taking the use as an absorbent article into consideration.

Further, the water-absorbent sheet of the present invention has one feature in that a liquid has smaller liquid leakage, and the water-absorbent sheet has a leakage index of preferably 100 or less, more preferably 50 or less, and even more preferably 30 or less, when taking the use as an absorbent article into consideration.

Further, since the water-absorbent sheet of the present invention has a very small used amount of a material derived from nature, consideration has been made to the environment while having high performance in thickness, permeation rate, and a leakage index as mentioned above. The proportion of the natural material is preferably 30% or less, more preferably 20% or less, and even more preferably 15% or less. The proportion of the natural material is calculated by dividing a total content of pulp, cotton and the like contained in very small amounts as the constituents of the water-absorbent sheet by mass of the water-absorbent sheet.

A water-absorbent sheet satisfying all the properties as mentioned above is very highly preferable in consideration of its use as an absorbent article.

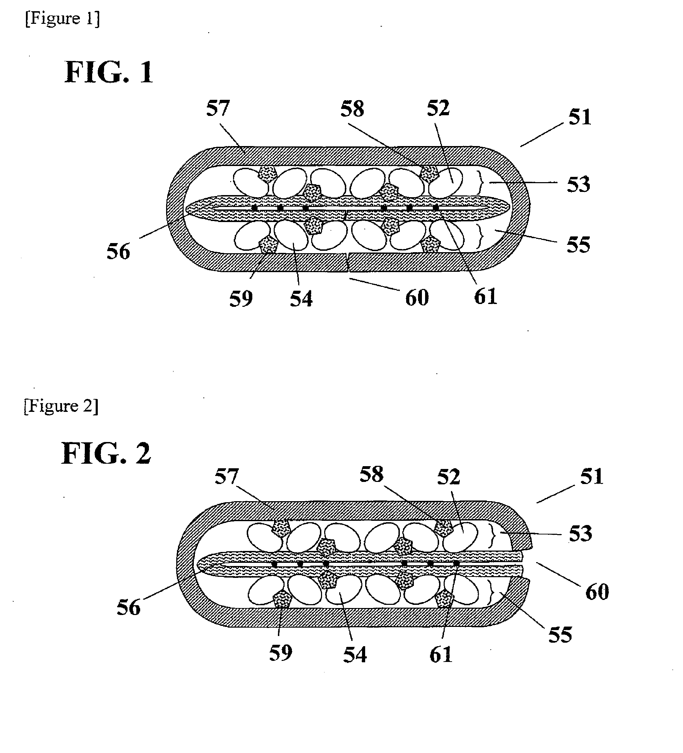

Next, the structure of the water-absorbent sheet of the present invention will be explained by referring to Figures 1 and 2. Here, Figures 1 and 2 are an enlarged cross-sectional view each schematically showing the structure of one embodiment of a water-absorbent sheet of the present invention.

A water-absorbent sheet 51 shown in Figure 1 comprises a primary absorbent layer 53 containing a water-absorbent resin 52 and an adhesive 58, and a secondary absorbent layer 55 containing a water-absorbent resin 54 and an adhesive 59. Here, the primary absorbent layer refers to a side to which a liquid to be absorbed is supplied upon the preparation of an absorbent article using the water-absorbent sheet, and the secondary absorbent layer refers to a side opposite to the primary absorbent layer in the state that an edge of a tip of the water absorbent sheet precursor is confronted in parallel to an edge of to other end of the tip, or in the state that the edges are adjoined to each other. At this time, a juncture 60 forming from edges of both the tips is formed. In the present invention, it is preferable that the juncture 60 is closed up with an adhesive or the like so that channels are not formed, or that the juncture 60 is provided on a side opposing a side to which a liquid is supplied. In the water absorbent sheet 51 shown in Figure 1, a side on which the juncture 60 is formed would be a secondary absorbent layer, and an opposing side sandwiching a substrate layer would be a primary absorbent layer. Here, the substrate layers are appropriately adhered with an adhesive 61 between the layers to form a two layer structure.

Moreover, a primary absorbent layer 53 and a secondary absorbent layer 55 are fractionated by a substrate layer 56 laminated in two layers, and a water absorbent sheet 51 has a 6 layer structure, comprising a primary absorbent layer 53, a secondary absorbent layer 55, a substrate layer 56 laminated in two layers, and front and back two layers made of a hydrophilic nonwoven fabric 57 positioned at each of the outer surface of the primary absorbent layer 53 and the secondary absorbent layer 55, which is a structure in which the absorbent layers are sandwiched by the hydrophilic nonwoven fabrics 57.

A water absorbent sheet 51 shown in Figure 2 comprises a primary absorbent layer 53 containing a water absorbent resin 52 and an adhesive 58, and a secondary absorbent layer 55 containing a water absorbent resin 54 and an adhesive 59. In the water absorbent sheet 51 shown in Figure 2, 58, and a secondary absorbent layer 55 containing a water-absorbent resin 54 and an adhesive 59. In the water-absorbent sheet 51 shown in Figure 2, the juncture 60 is formed in a side face of the water-absorbent 51, so that the setting of the primary absorbent layer and the secondary absorbent layer may be appropriately carried out irrespective to the position of the juncture 60.

Moreover, also in the water-absorbent sheet 51 shown in Figure 2, a primary absorbent layer 53 and a secondary absorbent layer 55 are fractionated by a substrate layer 56 laminated in two layers, in the same manner as the water-absorbent sheet shown in Figure 1. A water-absorbent sheet 51 has a 6-layer structure, comprising a primary absorbent layer 53, a secondary absorbent layer 55, a substrate layer 56 laminated in two layers, and front and back two layers made of a hydrophilic nonwoven fabric 57 positioned at each of the outer surface of the primary absorbent layer 53 and the secondary absorbent layer 55, which is a structure in which the absorbent layers are sandwiched by the hydrophilic nonwoven fabrics 57. Here, the substrate layer is fixed with an adhesive 61, and forms a two-layer structure.

Although the reasons why the substrate layer of the above-mentioned constitution serves to remarkably enhance the absorption capacities are not elucidated, they are deduced to be as follows. A liquid resides in an appropriate gap formed between the layers of the substrate, so that an appropriate balance between diffusion and permeation is likely to be obtained; therefore, the primary absorbent layer and the secondary absorbent layer can be effectively used, and as a result, a fast liquid permeation, a small amount of rewet and a small amount of a liquid leakage can be accomplished while avoiding the gel blocking phenomenon.

The water-absorbent sheet of the present invention can be produced by a method, for example, as described hereinbelow.

Specifically, the method for producing a water-absorbent sheet includes the steps of:

preparing a water-absorbent sheet precursor comprising an absorbent layer containing a water-absorbent resin and an adhesive, sandwiched between a substrate having a breathability in a front face side and a hydrophilic nonwoven fabric in a back face side; and

folding the water-absorbent sheet precursor in the front face side, and adhering the substrate formed in two layers with each other with an adhesive in an amount of from 0.1 to 50 g/m$^2$, thereby giving a water-absorbent sheet comprising a substrate layer having a total basis weight of the substrate is 25 g/m$^2$ to 250 g/m$^2$ formed thereon, the substrate layer being formed by laminating two or more layers of a substrate, wherein the water-absorbent sheet comprising an absorbent layer separated into a primary absorbent layer and a secondary absorbent layer with the substrate layer; wherein the substrate is made of at least one member selected from the group consisting of sanitary paper, porous films, cellulose-containing synthetic fiber nonwoven fabric, rayon-containing synthetic fiber nonwoven fabric, and hydrophilically treated synthetic fiber nonwoven fabric.

The method for producing a water-absorbent sheet of the present invention will be explained referring to the drawings.

(a) A mixed powder of a water-absorbent resin and an adhesive is evenly dispersed over regions A and B of a hydrophilic nonwoven fabric shown in Figure 3, and substrates constituting the substrate layer from the top are overlaid thereto. The overlaid substrates are subjected to heat-and-pressure fusing near a melting temperature of an adhesive, and unnecessary parts (edge side) are cut off along the line L. Next, an appropriate amount of an adhesive is dispersed on the side of the substrates, optionally heated to soften, and cooled, to hold the adhesive on the substrates. This water-absorbent sheet precursor is folded along the line K shown in Figure 3, so that a substrate side would be situated at an inner side, and the entirety thereof is again subjected to heat-and pressure fixing.

(b) A heat-fused adhesive is melt-coated on regions A and B of a hydrophilic nonwoven fabric shown in Figure 3, and thereafter a water-absorbent resin is evenly dispersed to form a layer, thereby holding the water-absorbent resin. Substrates produced by coating an adhesive previously heated and fused are overlaid from the top, optionally subjected to heat-and pressure fusing, and unnecessary parts (edge side) are cut off along the line L. Next, an appropriate amount of a heat-fused adhesive is applied to a substrate side, to give a water-absorbent sheet precursor. The above-mentioned water-absorbent sheet precursor is folded along the line K shown in Figure 3, so that the substrate side would be situated at an inner side, and again optionally heated and the entirety thereof is pressed.

(c) A mixed powder of a water-absorbent resin and an adhesive is evenly dispersed over regions C and D of a hydrophilic nonwoven fabric shown in Figure 4, and substrates constituting the substrate layer from the top are overlaid thereto. The overlaid substrates are subjected to heat-and-pressure fusing near a melting temperature of an adhesive, and unnecessary parts (edge side) are cut off along the line N. Next, an appropriate amount of an adhesive is dispersed on an upper part of the side of the substrates, and optionally heated to soften, to hold the adhesive on the substrates. This water-absorbent sheet precursor is folded along the line M shown in Figure 4, so that a substrate side would be situated at an inner side, and the entirety thereof is again subjected to heat-and-pressure fixing.

(d) A heat-fused adhesive is melt-coated on regions C and D of a hydrophilic nonwoven fabric shown in Figure 4, and thereafter a water-absorbent resin is evenly dispersed, thereby holding the water-absorbent resin. Substrates constituting the substrate layer, produced by coating an adhesive previously heated and fused are overlaid, optionally subjected to heat-and pressure fusing, and unnecessary parts (edge side) are cut off along the line N. Next, an appropriate amount of a heat-fused adhesive is applied to an upper part of a substrate side, to give a water-absorbent sheet precursor. The above-mentioned water-absorbent sheet precursor is folded along the line M shown in Figure 4, so that a substrate side would be situated at an inner side, and again optionally heated and the entirety thereof is pressed.

(e) Two sheets of substrates having breathability were pasted together with an adhesive to prepare a substrate layer having a specified constitution. Separately, a mixed powder of a water-absorbent resin and an adhesive is evenly dispersed over a hydrophilic nonwoven fabric, the above-mentioned substrate layer is further overlaid thereto, and the overlaid layers are subjected to heat-and-pressure fusing near a melting temperature of an adhesive. The mixed powder is dispersed on this water-absorbent sheet precursor in the same manner as the above, and the hydrophilic nonwoven fabric is again overlaid thereto, and subjected to heat-and-pressure fusing.

Among them, in the method (e), although a substrate layer having a special constitution of the present invention is used, in a case where typical procedures including the steps of laminating, on an upper part of a hydrophilic nonwoven fabric, an absorbent layer, a substrate layer, an absorbent layer, and finally a hydrophilic nonwoven fabric in that order, and subjecting to a heating process for retaining the shape between the laminates are appropriately carried out, the steps would be undesirably very complicated. In addition, there is a risk of making the balance between diffusion and permeation of the substrate layer unstable by repeats of heat-and-pressure to the substrate layer.

In other words, the methods (a) to (d) are preferably used, from the viewpoint of making the steps for obtaining a substrate layer having the constitution of the present invention efficient, and having a smaller variance in the properties of the water-absorbent sheet obtained. Among them, the methods (b) and (d) are more preferably used, from the viewpoint of being capable of more efficiently speeding up the production speed. The method of using an adhesive may be a combined use of a method of mixing powder with a water-absorbent resin, and a method of coating a heat-fused adhesive between a water-absorbent resin and a hydrophilic nonwoven fabric. Here, in the methods of (a) to (d), it is preferable that an area of an unnecessary part (edge side) is smaller, from the viewpoint of economical advantage.

When a water-absorbent sheet is folded and produced, a water- the water absorbent resin is likely to be suppressed, so that it is preferable that a non existing region (swellable margin) of a water absorbent resin is provided in the neighborhood of the fold. A water absorbent sheet prepared by providing a non existing region as described above would have a non existing region of a water absorbent resin along a longitudinal direction. The water absorbent sheet as described above is more preferred from the viewpoint of having a spatial room in an inner part of the water absorbent sheet even when the water absorbent sheet absorbs a body fluid or the like, to swell the water absorbent resin, thereby making it likely to exhibit the absorption properties inherently owned by the sheet without inhibiting the swelling of the water absorbent resin, and from the viewpoint of having a smaller loss of a water absorbent resin from a water absorbent sheet in both cases of during drying or wetting of the water absorbent sheet.

When a water absorbent sheet is prepared by folding a water absorbent sheet precursor, an edge of the tip of a water absorbent sheet precursor facing in parallel with an edge of the tip of the other end, or being in adjoining state. At this time, a juncture formed from edges of both the tips is formed. In the juncture, it is preferable that the edges of both the tips are in perfect contact with each other. When the bonding at a juncture is insufficient and channels are formed, a liquid leakage is likely to take place along the channels, for example, during slanting, when a liquid to be absorbed is supplied. Therefore, in the juncture, it is preferable to employ a method of completely subjecting to stopgap with an additive or the like so that channels are not formed in the juncture, and a preferable to employ a method of completely subjecting to stopgap with an additive or the like so that channels are not formed in the juncture, and a method of placing a juncture at a side to which a liquid to be absorbed is supplied, in other words, a side of a liquid-permeable sheet of an absorbent article. The latter method is more preferred, from the viewpoint of being a simpler production of an absorbent article.

The absorbent article of the present invention has a structure in which a water-absorbent sheet of the present invention is sandwiched between a liquid-permeable sheet and a liquid-impermeable sheet. The absorbent article includes, for example, disposable diapers, incontinence pads, sanitary napkins, pet sheets, drip sheets for foods, water blocking materials for electric power cables, and the like. Further, as the liquid-permeable sheet and the liquid-impermeable sheet, known ones in the technical field of the absorbent articles can be used without particular limitations. The absorbent article can be produced by a known method.

EXAMPLES

The present invention will be specifically described hereinbelow by the Examples, without intending to limit the scope of the present invention thereto.
The properties of the water-absorbent resin and the water-absorbent sheet were measured in accordance with the following methods.

< Water-Retention Capacity of Saline Solution of Water-Absorbent Resin >

The amount 2.0 g of water-absorbent resin was weighed in a cotton bag (Cottonbroad No. 60, width 100 mm × length 200 mm), and placed in a 500 mL-beaker. Physiological saline (0.9% by mass aqueous solution of sodium chloride, hereinafter referred to the same) was poured into the cotton bag in an amount of 500 g at one time, and the physiological saline was dispersed so as not to cause an unswollen lump of the water-absorbent resin. The upper part of the cotton bag was tied up with a rubber band, and the cotton bag was allowed to stand for 1 hour, to sufficiently make the water-absorbent resin swell. The cotton bag was dehydrated for 1 minute with a dehydrator (manufactured by Kokusan Enshinki Co., Ltd., product number: H-122) set to have a centrifugal force of 167G. The mass Wa (g) of the cotton bag containing swollen gels after the dehydration was measured. The same procedures were carried out without adding water-absorbent resin, and the empty mass Wb (g) of the cotton bag upon wetting was measured. The water-retention capacity of saline solution of the water-absorbent resin was calculated from the following formula.

$$\text{Water-Retention Capacity of Saline Solution (g/g) of}$$

$$\text{Water-Absorbent Resin}$$

$$= [Wa - Wb] \text{ (g)/Mass (g) of Water-Absorbent Resin}$$

. < Water Absorption Rate of Saline Solution of Water-Absorbent Resin >

This test was conducted in a room temperature-controlled to 25° ± 1°C. The amount 50 ± 0.1 g of physiological saline was weighed out in a 100 mL beaker, and a magnetic stirrer bar (8 mmφ × 30 mm, without a ring) was placed therein. The beaker was immersed in a thermostat, of which liquid temperature was controlled to 25° ± 0.2°C. Next, the beaker was placed over the magnetic stirrer so that a vortex was generated in physiological saline at a rotational speed of 600 r/min, the water-absorbent resin was then quickly added in an amount of 2.0 ± 0.002 g to the above beaker, and the time period (seconds) from a point of addition of the water-absorbent resin to a point of convergence of the vortex of the liquid surface was measured with a stopwatch, which was defined as a water absorption rate of the water-absorbent resin.

< Median Particle Size of Water-Absorbent Resin >

Unless specified otherwise, the particle size of the water-absorbent resin is defined as a median particle size, and measured as follows. An amorphous silica (Sipemat 200, Degussa Japan) was mixed in an amount of 0.5 g as a lubricant with 100 g of a water-absorbent resin.
The above-mentioned water-absorbent resin was allowed to pass though a JIS standard sieve having a sieve opening of 250 μm, and a median particle size was measured using a combination of sieves of (A) in a case where the resin is allowed to pass in an amount of 50% by mass or more, or a combination of sieves of (B) in a case where 50% by mass or more of the resin remains on the sieve.

(A) JIS standard sieves, a sieve having an opening of 425 μm, a sieve having an opening of 250 μm, a sieve having an opening of 180 μm, a sieve having an opening of 150 μm, a sieve having an opening of 106 μm, a sieve having an opening of 75 μm, a sieve having an opening of 45 μm, and a receiving tray were combined in order from the top.
(B) JIS standard sieves, a sieve having an opening of 850 μm, a sieve having an opening of 600 μm, a sieve having an opening of 500 μm, a sieve having an opening of 425 μm, a sieve having an opening of 300 μm, a sieve having an opening of 250 μm, a sieve having an opening of 150 μm, and a receiving tray were combined in order from the top.

The above-mentioned water-absorbent resin was placed on an uppermost sieve of the combined sieves, and shaken for 20 minutes with a rotating and tapping shaker machine to classify the resin.

After classification, the relationships between the opening of the sieve and an integral of a mass percentage of the water-absorbent resin remaining on the sieve were plotted on a logarithmic probability paper by calculating the mass of the water-absorbent resin remaining on each sieve as a mass percentage to an entire amount, and accumulating the mass percentages in order, starting from those having larger particle diameters. A particle diameter corresponding to a 50% by mass cumulative mass percentage is defined as a median particle size by joining the plots on the probability paper in a straight line. Here, the median particle size of the adhesive was obtained in the same manner as that of the water-absorbent resin, unless specified otherwise.

< Measurement of Thickness of Water-Absorbent Sheet >

The thickness of the resulting water-absorbent sheet was measured using a thickness measurement instrument (manufactured by Kabushiki Kaisha Ozaki Seisakusho, model number: J-B). As the measurement sites, three sites were arbitrarily determined in a longitudinal direction, on the left end, the center, and the right end; for example, in a water-absorbent sheet of 10 cm × 30 cm, the left end was set at a site 3 cm away from the left side, the center was set at a site 15 cm away therefrom, and the right end was set at a site 27 cm away therefrom. As the width direction, a uniform central part was measured.

The measurement value for thickness was obtained by measuring three times at each site and averaging the values for each site. Further, the values at the left end, the center, and the right end were averaged, to give a thickness of an overall water-absorbent sheet.

< Strength of Water-Absorbent Sheet >

The strength of the water-absorbent sheet was evaluated in accordance with the following method.

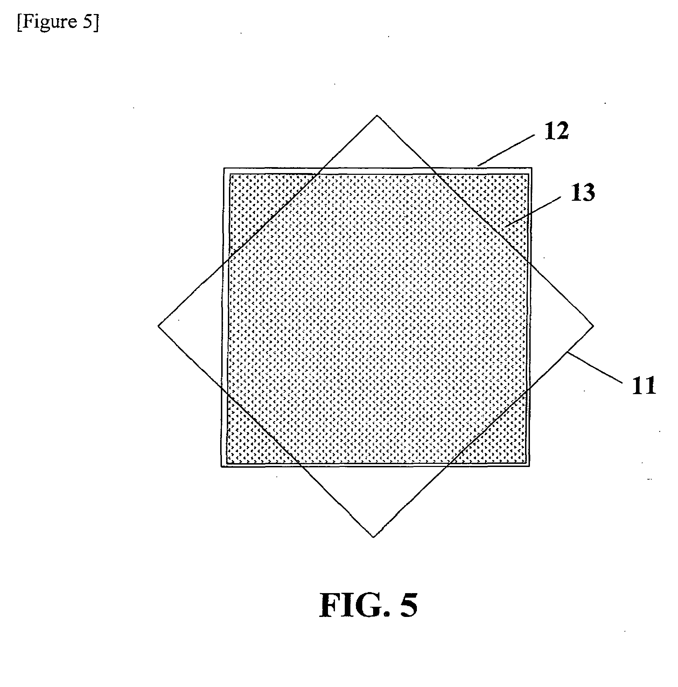

The resulting water-absorbent sheet was cut into a size of 10 cm × 10 cm. Next, the entire side of each of one side of two pieces of acrylic plates of 10 cm × 10 cm (mass about 60g) was adhered with a double-sided adhesive tape. As shown in Figure 5, the acrylic plates were pressed in a manner that the diagonal lines of acrylic plates 11, 12 form 45 degrees in angle, sandwiching from top and bottom to fix so that the double-sided adhesive tape faces the side of the water-absorbent sheet 13.

The strength-test pieces of the water-absorbent sheet prepared in the manner as described above were placed on a metallic tray of sieves, used in the section of the above-mentioned < Median Particle Size of Water-Absorbent Resin >, and a lid was put thereon. Thereafter, the lidded vessel was tapped with rotations with a rotating and tapping shaker machine for 3 minutes (at this time, a few layers of mesh sieves may be provided as a spacer between the tray and the tapping machine). The strength of the water-absorbent sheet was evaluated based on the external appearance after tapping in accordance with the following criteria.

○: The water-absorbent sheet showed no changes in external appearance, and did not easily move even when the acrylic plates were tried to be displaced.

△: The water-absorbent sheet showed no changes in external appearance, but the water-absorbent sheet was removed from the center when the acrylic plates were displaced.

×: The water-absorbent sheet was split in two from the center, and the contents were scattered.

[Evaluation of Absorbent Articles]

A simple water-absorbent article was prepared using the resulting water-absorbent sheet, and its physical properties were measured. The preparation conditions at this time were set to have the same conditions for all the water-absorbent sheets in Examples and Comparative Examples.

< Evaluations of Total Permeation Rate and Amount of Re-wet of Water-Absorbent Sheet >

A rectangular strip of a water-absorbent sheet of 10 × 30 cm, cut in a manner that a longitudinal direction thereof is to be in a length direction (machine feeding direction) of the hydrophilic nonwoven fabric, was used as a sample.

In a 10 L vessel were placed 60 g of sodium chloride, 1.8 g of calcium chloride dihydrate, 3.6 g of magnesium chloride hexahydrate, and a proper amount of distilled water to completely dissolve. Next, 15 g of an aqueous 1% by mass poly(oxyethylene) isooctylphenyl ether solution was added thereto, and distilled water was further added to adjust the weight of the overall aqueous solution to 6000 g. Thereafter, the mixed solution was colored with a small amount of Blue No. 1 to prepare a test solution.

On an upper part of a sample (water-absorbent sheet) was placed a polyethylene air-through style porous liquid-permeable sheet having the same size as the sample (10 × 30 cm) and a basis weight of 22 g/m$^2$. In addition, underneath the sample was placed a polyethylene liquid-impermeable sheet having the same size and basis weight as the sheet, to prepare a simple body liquid-absorbent article. A cylindrical cylinder having an inner diameter of 3 cm was placed near the central section of this body liquid-absorbent article, and a 50 mL test solution was supplied thereto at one time. At the same time, a time period until the test solution was completely permeated into the body liquid-absorbent article was measured with a stopwatch, which is referred to as a first permeation rate (sec). Next, the same procedures were carried out 30 minutes thereafter and 60 minutes thereafter, to measure second and third permeation rates (sec). A total of the number of seconds for the first to third permeation rates is referred to as a total permeation rate.

After 120 minutes from the start of the feeding of the first test liquid, the cylinder was removed, filter papers of 10 cm each side, of which mass was previously measured (Wg (g), about 70 g), were stacked near the liquid feeding position of the body liquid-absorbent article, and a 5 kg weight having a size of 10 cm × 10 cm was placed thereon. After 5 minutes of applying a load, the mass (Wh (g)) of the filter papers was measured, and an increased mass was defined as the amount of re-wet (g) as follows.

$$\text{Amount of Re-wet (g)} = \text{Wh} - \text{Wg}$$

< Slope Leakage Test >

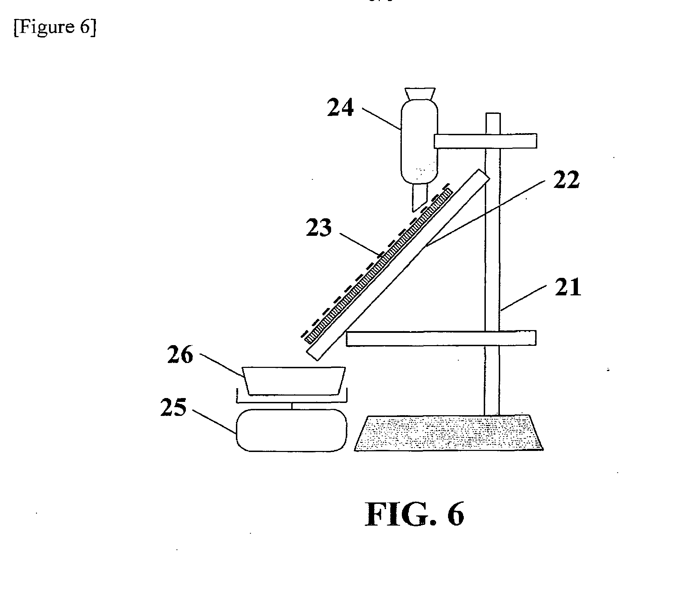

A slope leakage test was conducted using an apparatus shown in Figure 6.

Schematically, a mechanism is as follows. A commercially available stand 21 for experimental facilities was used to slope an acrylic plate 22 and fixed, the above-mentioned test solution was then supplied to a water-absorbent sheet 23 placed on the acrylic plate from a dropping funnel 24 positioned vertically above the sheet, and a leakage amount was measured with a balance 25. The detailed specifications are given hereinbelow.

An acrylic plate 22 has a length in the direction of the slope plane of 45 cm, and fixed so that an angle formed with an experimental stand 21 against the horizontal is 45° ± 2°. The acrylic plate 22 had a width of about 100 cm and a thickness of about 1 cm, and plural water-absorbent sheets 23 could be concurrently measured. The acrylic plate 22 had a smooth surface, so that a liquid was not detained or absorbed to the plate.

A dropping funnel 24 was fixed at a position vertically above the sloped acrylic plate 22 using an experimental stand 21. The dropping funnel 24 had a volume of 100 mL, and an inner diameter of a tip end portion of about 4 mm$\phi$, and an aperture of the cock was adjusted so that a liquid was supplied at a rate of 8 mL/seconds.

A balance 25 on which a metallic tray 26 was placed was set at a lower part of the acrylic plate 22, and all the test solutions flowing down the acrylic plate was received as leakage, and its mass was recorded to the accuracy of 0.1 g.

A slope leakage test using an apparatus as described above was carried out in accordance with the following procedures. The mass of a water-absorbent sheet 23 cut into a size of a length of 30 cm and a width of 10 cm was measured, and an air through-style polyethylene liquid permeable nonwoven fabric (basis weight: 22 g/m$^2$) of the same size was attached from an upper part thereof, and further a polyethylene liquid impermeable nonwoven fabric having the same basis weight of the same size was attached from a lower part thereof to prepare a simple absorbent article. The simple absorbent article was adhered on the acrylic plate 22 (in order not to stop leakage intentionally, the bottom end of the water-absorbent sheet 23 was not adhered to the acrylic plate 22).

Marking was put on the water-absorbent sheet 23 at a position 2 cm away in a downward direction from a top end thereof, and a supplying inlet for the dropping funnel 24 was fixed so that the inlet was positioned at a distance 8 mm ± 2 mm vertically above the marking.

A balance 25 was turned on, and tared so that the indication was zero, and thereafter 80 mL of the above-mentioned test solution was supplied at one time to the dropping funnel 24. An amount of liquid poured into a metallic tray 26 after the test solution was allowed to flow over a sloped acrylic plate 22 without being absorbed into a water-absorbent sheet 23 was measured, and this amount of liquid is referred to a first leakage amount (mL). The numerical value for this first leakage amount (mL) is denoted as LW1.

Second and third test solutions were supplied in 10-minute intervals from the beginning of the first supply, and second and third leakage

Next, a leakage index was calculated in accordance with the following equation. The more the index approaches to zero, the smaller the leakage amount at a slope of a water absorbent sheet, especially an initial leakage amount, whereby it is judged to be an excellent water absorbent sheet.

$$\text{Leakage Index:} \quad L = LW1 \times 10 + LW2 \times 5 + LW3$$

(Example 1)

A roller spreader (manufactured by HASHIMA CO., LTD., SINTERACE M C) was charged at its supplying inlet with a mixture prepared by homogeneously mixing 55 parts by mass of an ethylene vinyl acetate copolymer (melting temperature: 95°C, median particle size: 200 $\mu$m) as an adhesive and 270 parts by mass of a sodium polyacrylate crosslinked product A (manufactured by Sumitomo Seika Co., Ltd., AQUAKEEP SA55SX II, median particle size: 360 $\mu$m; water absorption rate of saline solution: 42 seconds, water retention capacity of saline solution: 35 g g, simply referred as "Resin A" in the table) as a water absorbent resin. On the other hand, a nonwoven fabric A having a width of 30 cm (spun lace rayon, basis weight: 45 g m$^2$, thickness: 400 $\mu$m, rayon content: 100%) was spread over a conveyor at the bottom part of the spreader, and covered with a paper sheet on areas other than region A in Figure 3 (width: 10 cm, gap between the regions A and B: 2 cm). Next, the spreading roller and the bottom part conveyor were operated, thereby allowing the above mentioned mixture to evenly overlay only over the region A of the above mentioned nonwoven fabric at a basis weight of 325 gm$^2$. region A of the above-mentioned nonwoven fabric at a basis weight of 325 g/m$^2$.

This laminate was carefully spread over the conveyor of the roller spreader again so that the mixture would not move, and thereafter covered with a paper sheet on areas other than region B (width: 5 cm) in Figure 3. The spreading roller and the bottom part conveyor were operated, thereby allowing the above-mentioned mixture to evenly overlay only over the region B of the above-mentioned nonwoven fabric at a basis weight of 78 g/m$^2$.

The laminate obtained was pressed from a top part with a nonwoven fabric B (spun lace rayon-PET, basis weight: 35 g/m$^2$, thickness: 300 $\mu$m, rayon content: 70%, PET content: 30%) as a substrate, and further heat-fused with a thermal laminating machine (manufactured by HASHIMA CO., LTD., straight linear fusing press HP-600LF) set at 130°C to integrate, to give a water-absorbent sheet precursor.

The roller spreader was charged at its supplying inlet with an ethylene-vinyl acetate copolymer (melting temperature: 95°C, median particle size: 200 $\mu$m) as an adhesive. On the other hand, the water-absorbent sheet precursor was spread over a conveyor at the bottom part of the spreader, so that the nonwoven fabric B would be situated at an upper side. Next, the spreading roller and the bottom part conveyor were operated, thereby allowing the above-mentioned adhesive to evenly overlay immediately over the region A of the above-mentioned water-absorbent sheet precursor at a basis weight of 15 g/m$^2$. Further, a heating furnace (set temperature: 110°C) also provided to the above-mentioned roller spreader was allowed to pass with the conveyor, and thereafter cooled to room temperature, thereby holding the adhesive.

The water-absorbent sheet precursor held with an adhesive was cut off at both the ends of the water-absorbent sheet precursor along the line L in Figure 3, and further folded along the line K (approximate central line of the regions A and B) in a manner that the adhesive would be situated at an inner side thereof. Thereafter, the adhesive is heat-fused with a thermal laminating machine (manufactured by HASHIMA CO., LTD., straight linear fusing press HP-600LF) set at 100°C to integrate, to give a water-absorbent sheet. The cross section of the structure of the water-absorbent sheet obtained had a structure as schematically shown in Figure 1. Here, it was confirmed that the juncture of the water-absorbent sheet produced in the present example was in a state that both the edges of the tips of the water-absorbent sheet precursor were completely united so that channels would not be formed. In the following Examples and Comparative Examples, it was confirmed that channels are not formed in the juncture of the water-absorbent sheet, unless specified otherwise.

(Example 2)

A roller spreader (manufactured by HASHIMA CO., LTD., SINTERACE M/C) was charged at its supplying inlet with a mixture prepared by homogeneously mixing 55 parts by mass of a copolymer polyester (melting temperature: 80°C, median particle size: 150 $\mu$m) as an adhesive and 270 parts by mass of the sodium polyacrylate crosslinked product A as a water-absorbent resin. On the other hand, a nonwoven fabric C having a width of 30 cm (spun lace rayon-PET, basis weight: 50 g/m$^2$, thickness: 500 $\mu$m, rayon content: 70%, PET content: 30%) was spread over a conveyor at the bottom part of the spreader, and covered with a paper sheet on areas other than region C in Figure 4 (width: 10 cm, gap between the regions C and D: 2 cm). Next, the spreading roller and the bottom part conveyor were operated, thereby allowing the above-mentioned mixture to evenly overlay only over the region C of the above-mentioned nonwoven fabric at a basis weight of 325 g/m$^2$.

This laminate was carefully spread over the conveyor of the roller spreader again so that the mixture would not move, and thereafter covered with a paper sheet on areas other than region D (width: 10 cm) in Figure 4. Next, a roller spreader was charged at its supplying inlet with a mixture prepared by homogeneously mixing 55 parts by mass of a copolymer polyester (melting temperature: 80°C, median particle size: 150 $\mu$m) as an adhesive and 270 parts by mass of a sodium polyacrylate crosslinked product B (manufactured by Sumitomo Seika Co., Ltd., AQUAKEEP 10SH-PB, median particle size: 320 $\mu$m; water-absorption rate of saline solution: 3 seconds, water retention capacity of saline solution: 42 g/g, simply referred as "Resin B" in the table) as a water-absorbent resin. The spreading roller and the bottom part conveyor were operated, thereby allowing the above-mentioned mixture to evenly overlay only over the region D of the above-mentioned nonwoven fabric at a basis weight of 78 g/m$^2$.

The laminate obtained was pressed from a top part with the nonwoven fabric C as a substrate, and further heat-fused with a thermal laminating machine (manufactured by HASHIMA CO., LTD., straight linear fusing press HP-600LF) set at 100°C to integrate, to give a water-absorbent sheet precursor.

The above-mentioned water-absorbent sheet precursor was spread over a hot melt applicator (manufactured by HALLYS Corporation, Marshall 150) of which heating temperature was set at 150°C, and thereafter a styrene-butadiene-styrene block copolymer (SBS, softening point: 85°C) was coated as an adhesive to an area immediately above the region C on the side of the nonwoven fabric C at a basis weight of 4 g/m$^2$ and a line width of 100 $\mu$m as a substrate.

The water-absorbent sheet precursor coated with the adhesive was cut off at the outer sides of the water-absorbent sheet precursor along the line N (distance between the region C or D and the line N: 1 cm) in Figure 4, and further folded along the line M (approximate central line of the regions C and D) in a manner that the adhesive would be situated at an inner side thereof. Thereafter, the adhesive is heat-fused with a thermal laminating machine (manufactured by HASHIMA CO., LTD., straight linear fusing press HP-600LF) set at 100°C to integrate, to give a water-absorbent sheet. The cross section of the structure of the water-absorbent sheet obtained had a structure as schematically shown in Figure 2.

(Example 3)

The nonwoven fabric C having a width of 30 cm was spread over a hot melt applicator (manufactured by HALLYS Corporation, Marshall 150) of which heating temperature was set at 150°C, and thereafter a styrene-butadiene-styrene block copolymer (SBS, softening point: 85°C) was coated as an adhesive over the nonwoven fabric. The coating method includes the steps of coating the adhesive to the region C shown in Figure 4 at a basis weight of 20 g/m$^2$ and a line width of 100 $\mu$m, and thereafter coating the adhesive to the region D at a basis weight of 5 g/m$^2$ and a line width of 60 $\mu$m.

A roller spreader (manufactured by HASHIMA CO., LTD., SINTERACE M/C) was charged at its supplying inlet with the sodium polyacrylate crosslinked product A as a water-absorbent resin. On the other hand, the above-mentioned nonwoven fabric coated with the adhesive was spread over the conveyor at the bottom part of the spreader, and covered with a paper sheet on areas other than region C in Figure 4. Next, the spreading roller and the bottom part conveyor were operated, thereby allowing the above-mentioned sodium polyacrylate crosslinked product A to evenly overlay only over the region C of the nonwoven fabric at a basis weight of 270 g/m$^2$, to give a laminate.

Next, a roller spreader (manufactured by HASHIMA CO., LTD., SINTERACE M/C) was charged at its supplying inlet with the sodium polyacrylate crosslinked product B as a water-absorbent resin. On the other hand, the above-mentioned laminate was spread over the conveyor at the bottom part of the spreader, and covered with a paper sheet on areas other than region D in Figure 4. Next, the spreading roller and the bottom part conveyor were operated, thereby allowing the above-mentioned sodium polyacrylate crosslinked product B to evenly overlay only over the region D of the nonwoven fabric at a basis weight of 65 g/m$^2$, to give a laminate dispersed with the water-absorbent resin in the regions C and D.

The above-mentioned laminate was pressed from a top part with a nonwoven fabric B coated with the SBS (softening point: 85°C) according to the same method, basis weight and line width as above, and thereafter heat-fused with a thermal laminating machine (manufactured by HASHIMA CO., LTD., straight linear fusing press HP-600LF) set at 100°C to integrate, to give a water-absorbent sheet precursor.

The above-mentioned water-absorbent sheet precursor was spread over the hot melt applicator of which heating temperature was set at 150°C with the nonwoven fabric B situated at an upper side, and thereafter the SBS (softening point: 85°C) was coated as an adhesive immediately above the region C on the side of the nonwoven fabric B at a basis weight of 4g/m$^2$ and a line width of 100 $\mu$m as a substrate.

The water-absorbent sheet precursor coated with the adhesive was heat-sealed and then cut off at the outer sides of the water-absorbent sheet precursor along the line N in Figure 4, and further folded along the line M in a manner that the adhesive would be situated at an inner side thereof. Thereafter, the adhesive is heat-fused with a thermal laminating machine (manufactured by HASHIMA CO., LTD., straight linear fusing press HP-600LF) set at 100°C to integrate, to give a water-absorbent sheet.

(Example 4)

The same procedures as in Example 3 were carried out except that in Example 3, as shown in Table 1, the nonwoven fabric C was changed to the nonwoven fabric A as a hydrophilic nonwoven fabric, that the nonwoven fabric B was changed to the nonwoven fabric C as a substrate for forming a substrate layer, that the amount of the SBS used as an adhesive was changed to that shown in Table 1, and that the amount of the water-absorbent resin and/or the adhesive dispersed was changed to that shown in Table 1, to give a water-absorbent sheet.

(Example 5)

The same procedures as in Example 4 were carried out except that in Example 4, the SBS as an adhesive was changed to the material (SBS/SIS) and the amount used as shown in Table 1, and that the amount of the water-absorbent resin and/or the adhesive dispersed was changed to that shown in Table 1, to give a water-absorbent sheet. The details of the material are as shown hereinbelow.

SBS/SIS adhesive: a mixture of a styrene-butadiene block copolymer and a styrene-isoprene block copolymer, softening point: 92°C (heating temperature of hot melt applicator: 145°C).

The water-absorbent sheet obtained was cut into a given size to measure the properties of the water-absorbent sheet. The results are shown in Table 2.

(Comparative Example 1)

A roller spreader (manufactured by HASHIMA CO., LTD., SINTERACE M/C) was charged at its supplying inlet with a mixture prepared by homogeneously mixing 55 parts by mass of an ethylene-vinyl acetate copolymer (melting temperature: 95°C, median particle size: 200 $\mu$m) as an adhesive and 330 parts by mass of the sodium polyacrylate crosslinked product A as a water-absorbent resin. On the other hand, the nonwoven fabric A having a width of 30 cm was spread over a conveyor at the bottom part of the spreader. Next, the spreading roller and the bottom part conveyor were operated, thereby allowing the above-mentioned mixture to evenly overlay over the above-mentioned nonwoven fabric at a basis weight of 385 g/m$^2$.

The laminate obtained was pressed from a top part with the above-mentioned nonwoven fabric A, and further heat-fused with a thermal laminating machine (manufactured by HASHIMA CO., LTD., straight linear fusing press HP-600LF) set at 130°C to integrate, to give a water-absorbent sheet.

The water-absorbent sheet was cut into a given size to measure the properties of the water-absorbent sheet. The results are shown in Table 2.

(Comparative Example 2)

A roller spreader (manufactured by HASHIMA CO., LTD., SINTERACE M/C) was charged at its supplying inlet with a mixture prepared by homogeneously mixing 55 parts by mass of an ethylene-vinyl acetate copolymer (melting temperature: 95°C, median particle size: 200 $\mu$m) as an adhesive and 270 parts by mass of the sodium polyacrylate crosslinked product A as a water-absorbent resin. On the other hand, a nonwoven fabric A having a width of 30 cm was spread over a conveyor at the bottom part of the spreader. Next, the spreading roller and the bottom part conveyor were operated, thereby allowing the above-mentioned mixture to evenly overlay over the above-mentioned nonwoven fabric at a basis weight of 325 g/m$^2$.

The laminate obtained was pressed from a top part with a nonwoven fabric D (spun lace rayon-PET, basis weight: 70 g/m$^2$, thickness: 700 $\mu$m, rayon content: 50%, PET content: 50%) as a substrate, and thereafter heat-fused with a thermal laminating machine (manufactured by HASHIMA CO., LTD., straight linear fusing press HP-600LF) set at 130°C to integrate. The laminate was again spread over the conveyor of the roller spreader with the nonwoven fabric D situated at an upper side. The spreading roller and the bottom part conveyor were operated, thereby allowing the above-mentioned mixture to evenly overlay over the above-mentioned nonwoven fabric at a basis weight of 78 g/m$^2$.

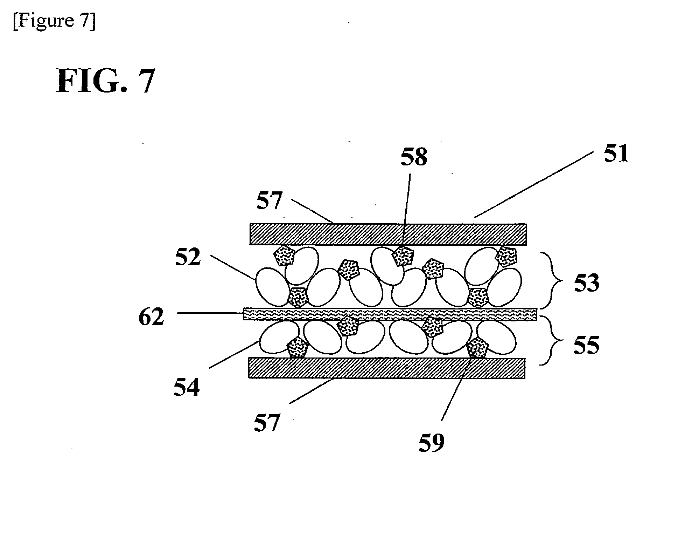

The laminate obtained was pressed from a top part with the above-mentioned nonwoven fabric A, and further heat-fused with a thermal laminating machine (manufactured by HASHIMA CO., LTD., straight linear fusing press HP-600LF) set at 130°C to integrate, to give a water-absorbent sheet. The cross section of the structure of the water-absorbent sheet obtained had a structure as schematically shown in Figure 7. Here, the substrate in the present example was different from a substrate layer in the inventive product and was composed of a single layer.

The water-absorbent sheet was cut into a given size to measure the properties of the water-absorbent sheet. The results are shown in Table 2.

(Comparative Examples 3 and 4)

The same procedures as in Example 3 except that in Example 3, as shown in Table 1, the nonwoven fabric B was changed to the nonwoven fabric C as a substrate for forming a substrate layer, that the amount of the adhesive used was changed to that shown in Table 1, and that the amount of the water-absorbent resin and/or the adhesive dispersed was changed to that shown in Table 1, to give a water-absorbent sheet.

(Comparative Example 5)

The same procedures as in Example 3 except that in Example 3, as shown in Table 1, the nonwoven fabric B was changed to a nonwoven fabric E (spun bond nonwoven fabric made of polyethylene terephthalate, basis weight: 13 g/m$^2$, PET content: 100%) as a substrate for forming a substrate layer, that the amount of the adhesive used was changed to that shown in Table 1, and that the amount of the water-absorbent resin and/or the adhesive dispersed was changed to that shown in Table 1, to give a water-absorbent sheet.

The water-absorbent sheet was cut into a given size to measure the properties of the water-absorbent sheet. The results are shown in Table 2.

[Table 1]

Table 1

| Example Number | Hydrophilic Nonwoven Fabric | Multiple Layered Substrate | | | | | | Water-Absorbent Resin $(g/m^2)$ | | | | |
|---|---|---|---|---|---|---|---|---|---|---|---|---|
| | Top/Bottom | Substrate | $g/m^2$ | Number of Substrates | Adhesive | $g/m^2$ | Primary | | Secondary | | Ratio* |
| Example 1 | Rayon | Rayon-PET | 35 | 2 | Ethylene-vinyl acetate | 15 | Resin A | 270 | Resin A | 65 | 81/19 |
| Example 2 | Rayon-PET | Rayon-PET | 50 | 2 | SBS | 4 | Resin A | 270 | Resin B | 65 | 81/19 |
| Example 3 | Rayon-PET | Rayon-PET | 35 | 2 | SBS | 4 | Resin A | 270 | Resin B | 65 | 81/19 |
| Example 4 | Rayon | Rayon-PET | 50 | 2 | SBS | 4 | Resin A | 200 | Resin B | 40 | 83/17 |
| Example 5 | Rayon | Rayon-PET | 50 | 2 | SBS/SIS | 8 | Resin A | 350 | Resin B | 150 | 70/30 |
| Comparative Example 1 | Rayon | - | - | - | - | - | Resin A | 330 | - | - | - |
| Comparative Example 2 | Rayon | Rayon-PET | 70 | 1 | - | - | Resin A | 270 | Resin A | 65 | 81/19 |
| Comparative Example 3 | Rayon-PET | Rayon-PET | 50 | 2 | SBS | 55 | Resin A | 270 | Resin B | 65 | 81/19 |
| Comparative Example 4 | Rayon-PET | Rayon-PET | 50 | 2 | SBS | 0.05 | Resin A | 270 | Resin B | 65 | 81/19 |
| Comparative Example 5 | Rayon-PET | PET | 13 | 2 | SBS | 3 | Resin A | 270 | Resin B | 65 | 81/19 |

-continued-

*: Ratio of primary absorbent layer to secondary absorbent layer, i.e. primary/secondary, of water-absorbent resin (mass ratio)

Primary absorbent layer: Region A (Figure 3), or Region C (Figure 4)

Secondary absorbent layer: Region B (Figure 3), or Region D (Figure 3)

-continued-

| Example Number | Adhesive (g/m²) | | | Content** | Location of Dispersion |
|---|---|---|---|---|---|
| | Kind | Primary | Secondary | | |
| Example 1 | Ethylene-vinyl acetate | 55 | 13 | 0.20 | Figure 3 |
| Example 2 | Polyester | 55 | 13 | 0.20 | Figure 4 |
| Example 3 | SBS | 40 | 10 | 0.15 | Figure 4 |
| Example 4 | SBS | 30 | 10 | 0.17 | Figure 4 |
| Example 5 | SBS/SIS | 50 | 20 | 0.14 | Figure 4 |
| Comparative Example 1 | Ethylene-vinyl acetate | 55 | - | 0.17 | - |
| Comparative Example 2 | Ethylene-vinyl acetate | 55 | 13 | 0.20 | - |
| Comparative Example 3 | SBS | 40 | 10 | 0.15 | Figure 4 |
| Comparative Example 4 | SBS | 40 | 10 | 0.15 | Figure 4 |
| Comparative Example 5 | SBS | 40 | 10 | 0.15 | Figure 4 |

**: Content of adhesive (content based on the water-absorbent resin (mass basis))
Primary absorbent layer: Region A (Figure 3), or Region C (Figure 4)
Secondary absorbent layer: Region B (Figure 3), or Region D (Figure 3)

[Table 2]

Table 2

| Example Number | Thickness | Permeation Rate (sec) | | | | Amount of Rewet | Slope Leakage Test | | | | Strength of Water-Absorbent Sheet |
|---|---|---|---|---|---|---|---|---|---|---|---|
| | (mm) | 1 | 2 | 3 | Total | (g) | 1 | 2 | 3 | Index | |
| Example 1 | 1.9 | 33 | 23 | 25 | 81 | 2.6 | 6 | 0 | 0 | 60 | ○ |
| Example 2 | 2.0 | 32 | 22 | 23 | 77 | 2.2 | 4 | 0 | 0 | 40 | ○ |
| Example 3 | 2.1 | 26 | 18 | 22 | 66 | 3.2 | 2 | 0 | 0 | 20 | ○ |
| Example 4 | 1.7 | 36 | 21 | 22 | 79 | 3.8 | 2 | 0 | 0 | 20 | ○ |
| Example 5 | 2.3 | 35 | 22 | 20 | 77 | 0.8 | 1 | 0 | 0 | 10 | ○ |
| Comparative Example 1 | 1.3 | 45 | 28 | 31 | 104 | 3.5 | 23 | 0 | 0 | 230 | Δ |
| Comparative Example 2 | 1.7 | 35 | 24 | 32 | 91 | 3.0 | 13 | 0 | 0 | 130 | ○ |
| Comparative Example 3 | 1.9 | 53 | 31 | 36 | 120 | 7.1 | 45 | 5 | 2 | 477 | ○ |
| Comparative Example 4 | 3.1 | 23 | 18 | 20 | 61 | 5.5 | 15 | 0 | 0 | 150 | × |
| Comparative Example 5 | 1.7 | 42 | 26 | 29 | 97 | 3.2 | 16 | 0 | 0 | 160 | Δ |

It could be seen from Tables 1 and 2 as follows. From the comparison between Examples 1 to 3 and Comparative Examples 1 to 5 in which the amount of the water-absorbent resin used is nearly the same, the water-absorbent sheets of the present invention using the substrate layers having the specified constitution had excellent properties in permeation rate, amount of re-wet, and slope leakage index. Further, even in the water-absorbent sheets of the present invention in Examples 4 and 5 having a modified amount of the water-absorbent resin used and a modified ratio of the primary absorbent layer/secondary absorbent layer used, the water-absorbent sheets had excellent properties in permeation rate, amount of re-wet, and slope leakage index.

(Example 6, Comparative Example 6)

Manufacture of Absorbent Articles

A back sheet side of a product manufactured by Procter and Gamble under the trade name Pampers Cottoncare (L size) was cut to open, and the contents were carefully removed so as not to destroy the top sheet. Each of the water-absorbent sheets obtained in Example 3 and Comparative Example 1 cut into pieces of 10 cm × 40 cm were inserted from the cut in a manner that a primary absorbent layer would be situated at the top sheet side, and sealed, to give absorbent articles (Example 6, Comparative Example 6). A test was conducted on 10 panelists using these absorbent articles. As a result, an evaluation was obtained that the absorbent article of Example 6 is more excellent in the aspect of feel, dry feel upon exchanging diapers, and liquid leakage.

It can be seen that the water-absorbent sheets of Examples 1 to 5 and the absorbent article of Example 6 are water-absorbent sheets and an absorbent article which accomplished thinning and avoidance of the gel blocking phenomenon even though the water-absorbent resin is contained in a large amount, while obtaining basic properties (strength, permeation rate, amount of re-wet, and amount of liquid leakage) at high levels.

INDUSTRIAL APPLICABILITY

The water-absorbent sheet of the present invention can be used for absorbent articles in hygienic material fields, agricultural fields, construction material fields, and the like, among which the water-absorbent sheet can be suitably used for absorbent articles in the hygienic material fields.

EXPLANATION OF NUMERICAL SYMBOLS

11   acrylic plate
12   acrylic plate
13   water-absorbent sheet
21   stand
22   acrylic plate
23   water-absorbent sheet
24   dropping funnel
25   balance
26   metallic tray
51   water-absorbent sheet
52   water-absorbent resin
53   primary absorbent layer
54   water-absorbent resin
55   secondary absorbent layer
56   substrate layer
57   hydrophilic nonwoven fabric
58   adhesive
59   adhesive
60   juncture
61   adhesive
62   substrate

**Claims**

1. A water-absorbent sheet 23 comprising a structure in which an absorbent layer comprising a water-absorbent resin and an adhesive is sandwiched with a hydrophilic nonwoven fabric 57, wherein the water-absorbent sheet has a structure in which the absorbent layer is fractionated into a primary absorbent layer 53 and a secondary absorbent layer 55 with a substrate layer formed by laminating two or more layers of a substrate having breathability 56 adhered together with an adhesive 61, the substrate layer having a basis weight of 25 g/m$^2$ to 250 g/m$^2$ and wherein the water-absorbent sheet is obtained by folding the front face side of a water-absorbent sheet precursor and adhering the substrate formed in two layers with each other with an adhesive in an amount of from 0.1 to 50 g/m$^2$, the water-absorbent sheet precursor comprising an absorbent layer comprising a water-absorbent resin and an adhesive, sandwiched between a substrate having a breathability in a front face side and a hydrophilic nonwoven fabric in a back face side;
   wherein the substrate is made of at least one member selected from the group consisting of sanitary paper, porous films, cellulose-containing synthetic fiber nonwoven fabric, rayon-containing synthetic fiber nonwoven fabric, and hydrophilically treated synthetic fiber nonwoven fabric.

2. The water-absorbent sheet according to claim 1, wherein the hydrophilic nonwoven fabric is made of at least one member selected from the group consisting of rayon fibers, polyolefin fibers, polyester fibers, and mixtures thereof.

3. The water-absorbent sheet according to claims 1 or 2, wherein the adhesive in the absorbent layer is at least one member selected from the group consisting of polyolefin-based adhesives, polyester-based adhesives, ethylene-vinyl acetate copolymer adhesives, and styrenic elastomer-based adhesives.

4. The water-absorbent sheet according to any one of claims 1 to 3, wherein the adhesive in the absorbent layer is contained in an amount of 0.05 to 2.0 times the amount of the water-absorbent resin contained (mass basis).

5. The water-absorbent sheet according to any one of claims 1 to 4, wherein the water-absorbent resin in the absorbent layer is contained in an amount of from 100 to 1000 g/m$^2$.

6. The water-absorbent sheet according to any one of claims 1 to 5, wherein the water-absorbent sheet comprises a non-existing region of a water-absorbent resin extending in a longitudinal direction of the sheet.

7. An absorbent article comprising the water-absorbent sheet as defined in any one of claims 1 to 6, sandwiched between a liquid-permeable sheet and a liquid-impermeable sheet.

8. A method for producing a water-absorbent sheet comprising the steps of:

   preparing a water-absorbent sheet precursor comprising an absorbent layer 53, 55 comprising a water-absorbent resin 52, 54 and an adhesive 58, sandwiched between a substrate having a breathability 56 in a front face side and a hydrophilic nonwoven fabric 57 in a back face side; and
   folding the water-absorbent sheet precursor in the front face side, and adhering the substrate formed in two layers with each other with an adhesive in an amount of from 0.1 to 50 g/m$^2$,
   thereby giving a water-absorbent sheet comprising a substrate layer having a total basis weight of the substrate of 25 g/m$^2$ to 250 g/m$^2$, the substrate layer being formed by laminating two or more layers of a substrate, wherein the water-absorbent sheet comprises an absorbent layer separated into a primary absorbent layer 53 and a secondary absorbent layer 55 with the substrate layer;

   wherein the substrate is made of at least one member selected from the group consisting of sanitary paper, porous films, cellulose-containing synthetic fiber nonwoven fabric, rayon-containing synthetic fiber nonwoven fabric, and hydrophilically treated synthetic fiber nonwoven fabric.

**Patentansprüche**

1. Wasserabsorbierende Bahn 23, umfassend eine Struktur, in der eine Absorptionsschicht, umfassend ein wasserabsorbierendes Harz und ein Haftmittel, von einem hydrophilen Textilverbundstoff 57 sandwichartig umgeben ist, wobei die wasserabsorbierende Bahn eine Struktur aufweist, in der die Absorptionsschicht in eine primäre Absorptionsschicht 53 und in eine sekundäre Absorptionsschicht 55 durch eine Substratschicht getrennt ist, gebildet durch Laminieren zweier oder mehrerer Schichten eines Substrats mit Atmungsaktivität 56, durch ein Haftmittel 61 zusammenhaftend, wobei die Substratschicht ein Basisgewicht von 25 g/m$^2$ bis 250 glm$^2$ aufweist, und wobei die wasserabsorbierende Bahn erhalten ist durch Falten der Vorderseite eines wasserabsorbierenden Bahnvorläufers und miteinander Verkieben des in zwei Schichten gebildeten Substrats mit einem Haftmittel in einer Menge von 0,1 bis 50 g/m$^2$, wobei der wasserabsorbierende Bahnvorläufer eine Absorptionsschicht, umfassend ein wasserabsorbierendes Harz und ein Haftmittel, sandwichartig zwischen einem Substrat mit einer Atmungsaktivität auf einer Vorderseite und einem hydrophilen Textilverbundstoff auf einer Rückseite angeordnet, umfasst;

wobei das Substrat aus mindestens einem Bestandteil, ausgewählt aus der Gruppe, bestehend aus Hygienepapier, porösen Filmen, Cellulose-haltigem Kunstfasertextilverbundstoff, Rayon-haltigem Kunstfasertextilverbundstoff und hydrophil behandeltem Kunstfasertextilverbundstoff, hergestellt ist.

2.  Wasserabsorbierende Bahn nach Anspruch 1, wobei der hydrophile Textilverbundstoff aus mindestens einem Bestandteil, ausgewählt aus der Gruppe, bestehend aus Rayonfasern, Polyolefinfasern, Polyesterfasern und Gemischen davon, hergestellt ist.

3.  Wasserabsorbierende Bahn nach Anspruch 1 oder 2, wobei das Haftmittel in der Absorptionsschicht mindestens ein Bestandteil, ausgewählt aus der Gruppe, bestehend aus polyolefinbasierten Haftmittein, polyesterbasierten Haftmitteln, Ethylen-Vinylacetat-Copolymer-Haftmitteln und styrolischen elastomerbasierten Haftmitteln, ist.

4.  Wasserabsorbierende Bahn nach einem der Ansprüche 1 bis 3, wobei das Haftmittel in der Absorptionsschicht in einer Menge der 0,05- bis 2,0-fachen Menge (massenbasiert) des enthaltenen wasserabsorbierenden Harzes enthalten ist.

5.  Wasserabsorbierende Bahn nach einem der Ansprüche 1 bis 4, wobei das wasserabsorbierende Harz in der Absorptionsschicht in einer Menge von 100 bis 1000 g/m$^2$ enthalten ist.

6.  Wasserabsorbierende Bahn nach einem der Ansprüche 1 bis 5, wobei die wasserabsorbierende Bahn einen nicht vorhandenen Bereich eines wasserabsorbierenden Harzes, sich in einer Längsrichtung der Bahn erstreckend, umfasst.

7.  Absorbierender Gegenstand, umfassend die wasserabsorbierende Bahn nach einem der Ansprüche 1 bis 6, sandwichartig zwischen einer flüssigkeitsdurchlässigen Bahn und einer flüssigkeitsundurchlässigen Bahn angeordnet.

8.  Verfahren zur Herstellung einer wasserabsorbierenden Bahn, umfassend die Schritte:

    Bereitstellen eines wasserabsorbierenden Bahnvorläufers, umfassend eine Absorptionsschicht 53, 55, umfassend ein wasserabsorbierendes Harz 52, 54 und ein Haftmittel 58, sandwichartig zwischen einem Substrat mit einer Atmungsaktivität 56 auf einer Vorderseite und einem hydrophilen Textilverbundstoff 57 auf einer Rückseite angeordnet; und

    Falten des wasserabsorbierenden Bahnvorläufers auf der Vorderseite und miteinander Verkleben des in zwei Schichten gebildeten Substrats mit einem Haftmittel in einer Menge von 0,1 bis 50 g/m$^2$, dabei ergebend eine wasserabsorbierende Bahn, umfassend eine Substratschicht mit einem Gesamtbasisgewicht des Substrats von 25 g/m$^2$ bis 250 g/m$^2$, wobei die Substratschicht durch Laminieren zweier oder mehrerer Schichten eines Substrats gebildet ist, wobei die wasserabsorbierende Bahn eine Absorptionsschicht, getrennt in eine primäre Absorptionsschicht 53 und eine sekundäre Absorptionsschicht 55 durch die Substratschicht, umfasst;

    wobei das Substrat aus mindestens einem Bestandteil, ausgewählt aus der Gruppe, bestehend aus Hygienepapier, porösen Filmen, Cellulose-haltigem Kunstfasertextilverbundstoff, Rayon-haltigem Kunstfasertextilverbundstoff und hydrophil behandeltem Kunstfasertextilverbundstoff, hergestellt ist.

## Revendications

1.  Feuille hydro-absorbante 23 comprenant une structure dans laquelle une couche absorbante comprenant une résine hydro-absorbante et un adhésif est prise en sandwich dans un non-tissé hydrophile 57, dans laquelle la feuille hydro-absorbante présente une structure dans laquelle la couche absorbante est séparée entre une couche absorbante primaire 53 et une couche absorbante secondaire 55 avec une couche de substrat formée en laminant deux ou plusieurs couches d'un substrat présentant une respirabilité 56 collées les unes aux autres à l'aide d'un adhésif 61, la couche de substrat ayant un poids de base de 25 g/m$^2$ à 250 g/m$^2$ et dans laquelle la feuille hydro-absorbante est obtenue en pliant le côté face avant d'un précurseur de feuille hydro-absorbante et en collant l'une à l'autre les deux couches formant le substrat à l'aide d'un adhésif en une quantité de 0,1 à 50 g/m$^2$, le précurseur de feuille hydro-absorbante comprenant une couche absorbante comprenant une résine hydro-absorbante et un adhésif, prise en sandwich entre un substrat présentant une respirabilité côté face avant et un non-tissé hydrophile côté face arrière ;

dans laquelle le substrat est constitué d'au moins un élément choisi dans le groupe constitué du papier hygiénique, des films poreux, du non-tissé de fibres synthétiques contenant de la cellulose, du non-tissé de fibres synthétiques contenant de la rayonne et du non-tissé de fibres synthétiques traitées de manière à être hydrophiles.

2. Feuille hydro-absorbante selon la revendication 1, dans laquelle le non-tissé hydrophile est constitué d'au moins un élément choisi dans le groupe constitué des fibres de rayonne, des fibres de polyoléfine, des fibres de polyester et des mélanges de celles-ci.

3. Feuille hydro-absorbante selon les revendications 1 ou 2, dans laquelle l'adhésif de la couche absorbante est au moins un élément choisi dans le groupe constitué des adhésifs à base de polyoléfine, des adhésifs à base de polyester, des adhésifs à base de copolymère éthylène-acétate de vinyle et des adhésifs à base d'élastomères styréniques.

4. Feuille hydro-absorbante selon l'une quelconque des revendications 1 à 3, dans laquelle l'adhésif de la couche absorbante est contenu en une quantité de 0,05 à 2,0 fois la quantité de la résine hydro-absorbante contenue (base massique).

5. Feuille hydro-absorbante selon l'une quelconque des revendications 1 à 4, dans laquelle la résine hydro-absorbante de la couche absorbante est contenue en une quantité de 100 à 1 000 g/m$^2$.

6. Feuille hydro-absorbante selon l'une quelconque des revendications 1 à 5, dans laquelle la feuille hydro-absorbante comprend une région non existante d'une résine hydro-absorbante s'étendant dans une direction longitudinale de la feuille.

7. Article absorbant comprenant la feuille hydro-absorbante telle que définie dans l'une quelconque des revendications 1 à 6, prise en sandwich entre une feuille perméable aux liquides et une feuille imperméable aux liquides.

8. Procédé de production d'une feuille hydro-absorbante comprenant les étapes consistant à :

préparer un précurseur de feuille hydro-absorbante comprenant une couche absorbante 53, 55 comprenant une résine hydro-absorbante 52, 54 et un adhésif 58, prise en sandwich entre un substrat présentant une respirabilité 56 côté face avant et un non-tissé hydrophile 57 côté face arrière ; et

plier le précurseur de feuille hydro-absorbante côté face avant, et coller l'une à l'autre les deux couches formant le substrat à l'aide d'un adhésif en une quantité de 0,1 à 50 g/m$^2$,

pour ainsi obtenir une feuille hydro-absorbante comprenant une couche de substrat ayant un poids de base total du substrat de 25 g/m$^2$ à 250 g/m$^2$, la couche de substrat étant formée en laminant deux ou plusieurs couches d'un substrat, dans laquelle la feuille hydro-absorbante comprend une couche absorbante séparée entre une couche absorbante primaire 53 et une couche absorbante secondaire 55 avec la couche de substrat ;

dans laquelle le substrat est constitué d'au moins un élément choisi dans le groupe constitué du papier hygiénique, des films poreux, du non-tissé de fibres synthétiques contenant de la cellulose, du non-tissé de fibres synthétiques contenant de la rayonne et du non-tissé de fibres synthétiques traitées de manière à être hydrophiles.

[Figure 1]

# FIG. 1

[Figure 2]

# FIG. 2

[Figure 3]

# FIG. 3

[Figure 4]

# FIG. 4

[Figure 5]

**FIG. 5**

[Figure 6]

**FIG. 6**

[Figure 7]

# FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5895379 A
- US 2003163106 A
- FR 2364024
- US 2007197987 A
- JP HEI9510889 B
- JP HEI857311 B

- JP 2000238161 A
- JP 2003011118 A
- JP HEI02048944 B
- JP HEI6059039 B
- JP HEI9313530 B
- JP 2002345871 A